# EUROPÄISCHE PATENTSCHRIFT

(11) **EP 0 423 070 B1**
(45) Veröffentlichungstag und Bekanntmachung des Hinweises auf die Patenterteilung: **30.08.1995**
(21) Anmeldenummer: 90810762.6
(22) Anmeldetag: 04.10.1990
(51) Int. Cl.: C07C 37/50, C07C 39/06

(54) **Verfahren zur Herstellung substituierter Phenole**
Process for preparing substituted phenols
Procédé de préparation de phénols substitués

(30) Priorität: 12.10.1989 CH 3721/89
(43) Veröffentlichungstag der Anmeldung: 17.04.1991
(73) Patentinhaber: CIBA-GEIGY AG, 4002 Basel (CH)
(72) Erfinder: Illy, Hugo, Dr., CH-4153 Reinach (CH); Salathé, Ronald, CH-4312 Magden (CH); Schwabe, Rudolf, Dr., CH-4412 Nuglar (CH)

(56) Entgegenhaltungen:
- EP-A- 0 057 160
- GB-A- 1 494 823
- CHEMICAL ABSTRACTS, vol. 111, no. 10, 4. September 1989, Columbus, Ohio, US; abstract no. 80998E, GORODETSKII, E. T.; TRENDOVATSKII, P. I.; LAR'KOVA, V. M.: 'Dealkylation of 2,6-di-tert-butyl-4-methylphenol on hydrogen sulfates' Seite 180 ; & KINET. KATAL. Bd. 30, Nr. 1, Seiten 134 - 7;
- "Methoden der Organischen Chemie (Houben-Weyl), Band VI/c: PHENOLE Teil 2", GEORG THIEME VERLAG, STUTTGART

## Beschreibung

Die vorliegende Erfindung betrifft ein neues Verfahren zur Herstellung substituierter Phenole.

Phenole mit bestimmten Substitutionsmustern dienen häufig als Ausgangs- bzw. Zwischenprodukte für die Herstellung von Endprodukten unterschiedlichster Eigenschaften. Beispielsweise sind substituierte Phenole, die eine freie ortho-Position aufweisen, begehrte Ausgangsmaterialien für die Herstellung von UV-Absorbern vom Benztriazoltyp, wie dies z.B. in EP-A-57 160 beschrieben ist.

Solche Phenole werden üblicherweise durch Entalkylierung der ortho-Position substituierter Phenole hergestellt, welche in der Regel aus der teerverarbeitenden Industrie erhältlich sind, oder sie können z.B. durch Michael-Addition von olefinischen Verbindungen an entsprechende Phenole hergestellt werden. Dies ist z.B. in US-A-4,085,132 und US-A-4,228,297 beschrieben.

Die Entalkylierung bzw. Entbutylierung von substituierten Phenolen wurde beispielsweise schon von D.R. Stevens in Ind.Eng.Chem. 35, No. 6, 655 (1943) und G.H. Stillson et al in JACS 67, 305 (1945) am Beispiel o-butylierter Kresole gezeigt. Sie fanden, dass Schwefelsäure, Schwefelsäureester und aromatische Sulfonsäuren diese Entbutylierungen katalysieren. Die Entfernung von Methylgruppen aus den untersuchten o-butylierten Kresolen wurde dagegen nicht beobachtet. Ein ähnliches Ergebnis ist auch in GB-A-1,183,984 beschrieben, wo unter Verwendung von Fe₂(SO₄)₃·xH₂O die Entbutylierung von in ortho-Stellung t-butylsubstituierten Phenolen durchgeführt wird.

In Kinet. Katal. 30(1)1989,134-7 wird die Entalkylierung von 2,6-Di-tert.butyl-4-methylphenol mit auf einem Silikagel fixierten Hydrogensulfat als Katalysator beschrieben.

Diese Verfahren besitzen jedoch den Nachteil, dass sie bezüglich der häufig gewünschten Entalkylierung von nur einer einzigen ortho-Position nicht genügend selektiv verlaufen.

Es werden nämlich durchweg Produkte erhalten, die sich als Mischung von in einer ortho-Stellung und in beiden ortho-Stellungen entalkylierten Verbindungen erweisen. Diese Uneinheitlichkeit der Produkte verhindert in vielen Fällen ihre direkte Weiterverarbeitung und erfordert umständliche Auftrennungsprozesse zur Isolierung des gewünschten einfach dealkylierten Produkts.

Aufgabe der vorliegenden Erfindung ist es nun, ein Verfahren zur Herstellung substituierter Phenole vorzuschlagen, das bezüglich Entalkylierung eine höhere Selektivität aufweist als die obengenannten Verfahren und in hohen Ausbeuten zu in nur einer ortho-Stellung dealkylierten Phenolen führt.

Die Aufgabe wurde erfindungsgemäss gelöst, indem man zur Dealkylierung substituierter Phenole ein Alkalimetallhydrogensulfat als Katalysator verwendete.

Gegenstand der vorliegenden Erfindung ist somit ein Verfahren zur Herstellung von Phenolen der Formel 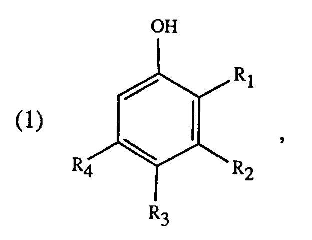
worin R₁, R₂, R₃ und R₄ unabhängig voneinander Wasserstoff, gegebenenfalls substituiertes Alkyl mit 1 bis 12 Kohlenstoffatomen oder gegebenenfalls substituiertes Alkenyl oder Alkinyl mit 2 bis 12 Kohlenstoffatomen sind, durch Erhitzen der Phenole der Formel 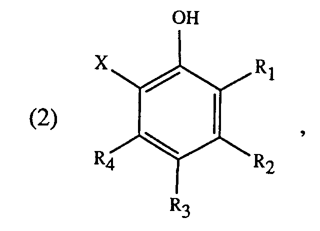
worin X ein sekundäres oder tertiäres Alkyl mit 3 bis 12 Kohlenstoffatomen ist, und R₁, R₂, R₃ und R₄ die angegebenen Bedeutungen haben, in Gegenwart eines sauren Katalysators, dadurch gekennzeichnet, dass man die Phenole der Formel (2) zur Abspaltung des Substituenten X mit Natriumhydrogensulfat als Katalysator, welcher nicht auf einem Trägermaterial fixiert ist, über ihren Schmelzpunkt erhitzt, die Schmelze abkühlen lässt und gegebenenfalls die Phenole der Formel (1) isoliert.

In den Phenolen der Formel (1) bedeuten die Substituenten R₁, R₂, R₃ und R₄ unabhängig voneinander Wasserstoff, gegebenenfalls substituiertes Alkyl mit 1 bis 12 Kohlenstoffatomen oder gegebenenfalls substituiertes Alkenyl oder Alkinyl mit je 2 bis 12 Kohlenstoffatomen. Geeignete Alkylreste sind somit beispielsweise Methyl, Aethyl, Propyl, Butyl, Hexyl, Octyl, Decyl und Dodecyl sowie entsprechende verzweigte Isomere. Diese Alkylreste können mit solchen Substituenten versehen sein, welche die erfindungsgemässe Umsetzung nicht beeinträchtigen, wie z.B. Hydroxyl, Alkoxy, Alkoxycarbonyl, -CO₂Z, worin Z Wassersotff, Alkyl mit 1 bis 18 Kohlenstoffatomen oder 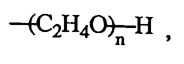
wobei n 1 bis 12 ist, ist, und Phenyl. Als Alkenylreste kommen u.a. Aethenyl, Propenyl, Butenyl, Pentenyl, Hexenyl, Octenyl und Dodecenyl in Frage, die entsprechenden verzweigten Isomere sowie Isomere, die mehr als eine Doppelbindung enthalten. Als Beispiele geeigneter Alkinylreste sind die den aufgezählten Alkenylresten analogen Gruppen zu nennen, also Aethinyl, Popinyl usw. Die Alkenyl und Alkinylreste können substituiert sein und z.B. die für die Alkylreste genannten Substituenten tragen.

Der Substituent X in den Phenolen der Formel (2) bedeutet sekundäres oder tertiäres Alkyl mit 3 bis 12 Kohlenstoffatomen, also z.B. i-Propyl, t-Butyl, i-Hexyl und i-Octyl, wobei t-Butyl eine bevorzugte Bedeutung von X ist.

Vorzugsweise werden im erfindungsgemässen Verfahren Phenole der Formel 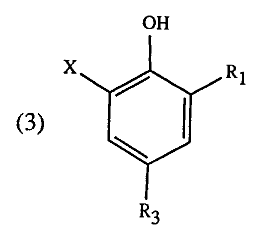
eingesetzt, worin R₁, R₃ und X die angegebenen Bedeutungen haben.

Von diesen Phenolen werden solche bevorzugt, worin R₁ Wasserstoff, Alkyl mit 1 bis 12 Kohlenstoffatomen, Benzyl, Phenyläthyl oder Phenylpropyl, insbesondere 2-Phenylpropyl, R₃ gegebenenfalls mit Phenyl, -CO₂Z, worin Z Wasserstoff, Alkyl mit 1 bis 18 Kohlenstoffatomen oder 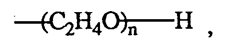
worin n 1 bis 12 ist, bedeutet, substituiertes Alkyl mit 1 bis 8 Kohlenstoffatomen und X tertiäres Alkyl mit 4 bis 9 Kohlenstoffatomen ist.

Hiervon besonders geeignete Phenole sind jene, worin R₃ Methyl, t-Butyl oder -CH₂CH₂CO₂Z ist, worin Z die angegebene Bedeutung hat.

Besonders bevorzugte Phenole entsprechen der Formel 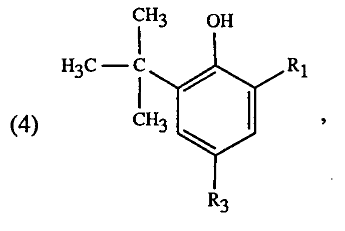
worin R₁ Wasserstoff oder Alkyl mit 1 bis 5 Kohlenstoffatomen, insbesondere Methyl oder t-Butyl und R₃ Alkyl mit 1 bis 4 Kohlenstoffatomen, insbesondere Methyl oder t-Butyl oder -CH₂CH₂CO₂CH₃ ist.

Der Substituent X lässt sich vom aromatischen System entfernen, wenn das betreffende Phenol der Formel (2) in Gegenwart eines Alkalimetallhydrogensulfats als Katalysator über seinen Schmelzpunkt erhitzt wird. Aus der erhaltenen Schmelze kann das entsprechende Phenol der Formel (1) auf an sich bekannte Weise, beispielsweise durch Filtration der heissen Schmelze, isoliert werden.

Natriumhydrogensulfat wird mit Vorteil in wasserfreier Form als Katalysator verwendet.

Neben der höheren Selektivität des Reaktionsverlaufs ist es ein weiterer Vorteil des erfindungsgemässen Verfahrens, dass für die meisten Zwecke auf die Isolierung des Produktes bzw. auf die Abtrennung des Katalysators und gegebenenfalls der Nebenprodukte verzichtet werden kann, da der Katalysator bei weiteren Umsetzungen nicht stört und Nebenprodukte nur in vernachlässigbar geringer Konzentration auftreten. Ferner ist das Reaktionsprodukt praktisch farblos und enthält keine durch die Abspaltung von X entstandenen Polymeren.

Das erfindungsgemässe Verfahren wird in der Regel so ausgeführt, dass ein Phenol der Formel (2) in einem evakuierbaren Reaktionsgefäss geschmolzen wird. Man gibt dann den Katalysator, vorzugsweise in Mengen von 0,5 bis 10 Mol%, insbesondere 1,5 bis 2 Mol%, bezogen auf eingesetztes Phenol, unter Rühren der Schmelze hinzu und kann dann die Temperatur für z.B. 2 bis 6 Stunden auf vorzugsweise 120 bis 170°C erhöhen. Vorzugsweise wird durch Reduzierung des Druckes im Reaktionsgefäss, z.B. auf Werte von 10 bis 300 mbar, entstehendes gasförmiges Abspaltungsprodukt entfernt. Man lässt die Schmelze dann abkühlen und erhält so ein Produkt, das direkt ohne weitere Reinigungsoperationen umgesetzt werden kann.

Zum Rühren der Schmelze eignen sich die bekannten Rührertypen, wie z.B. Anker-, Gitter-, Impeller- und insbesondere Ekatorührer.

Durch Austreiben der gasförmigen Abspaltungsprodukte aus der Schmelze mittels Stickstoffstrom oder einem siedenden, inerten Schleppmittel kann eine Beschleunigung des Reaktionsablaufes erzielt werden.

Die folgenden Beispiele erläutern die Erfindung. Prozentangaben beziehen sich auf das Gewicht.
Beispiel 1: In einem Reaktionsgefäss werden in einer Stickstoffatmosphäre bei 80°C 438 g 3-(3,5-Di-t-butyl-4-hydroxyphenyl)-propionsäuremethylester geschmolzen. Anschliessend werden 4,14 g Natriumhydrogensulfat zugegeben. Der Druck im Reaktionsgefäss wird auf 50 mbar eingestellt. Man erhitzt auf 150°C und hält die Temperatur dann während 4 Stunden konstant, wobei der Druck auf 50 mbar eingestellt bleibt. Nach Abkühlen der Schmelze auf Raumtemperatur ergibt die Analyse der Reaktionsmasse folgende Zusammensetzung:

| | |
|---|---|
| 3-(3,5-Di-butyl-4-hydroxyphenyl)-propionsäuremethylester | 3,8 % |
| 3-(3-t-Butyl-4-hydroxyphenyl)-propionsäuremethylester | 85,6% |
| Phlorethinsäuremethylester | 2,9 % |
| Dimere Verbindung | 6,0 % |

Nach der hydrolytischen Aufarbeitung beträgt die Reinheit des monobutylierten Produkts 92,0 % und der Schmelzpunkt 55 bis 60°C.
Beispiel 2: 396,6 g 2,6-Di-t-butyl-p-kresol werden in einer Stickstoffatmosphäre bei 150°C geschmolzen. Nach Zugabe von 4,97 g Natriumhydrogensulfat hält man die Reaktionsmischung während 3,5 Stunden bei 150°C, wobei der Druck im Reaktionsgefäss in einem Bereich von 20 bis 100 mbar eingestellt wird. Die Schmelze lässt man auf Raumtemperatur abkühlen. Die Analyse der Reaktionsmasse ergibt folgende Werte:

| | |
|---|---|
| 2,6-Di-t-butyl-p-kresol | 7,8 % |
| 2-t-butyl-p-kresol | 85,0 % |
| p-Kresol | 7,0 % |

Die Reinheit des monobutylierten Produktes beträgt 85 %, der Schmelzpunkt 47 bis 50°C.
Beispiel 3: Man geht wie in Beispiel 2 angegeben vor, setzt jedoch 396,6g 4,6-Di-t-butyl-o-kresol ein und hält nach Zugabe von 4,97 g Natriumhydrogensulfat die Reaktionsmischung während 4,5 Stunden bei 150°C. Nach Abkühlen auf Raumtemperatur besass die Reaktionsmasse die folgende Zusammensetzung:

| | |
|---|---|
| 4,6-Di-t-butyl-o-kresol | 2,0 % |
| 4-t-butyl-o-kresol | 97,0 % |
| o-Kresol | 0,6 % |

Die Reinheit des monobutylierten, bei Raumtemperatur flüssigen Produktes beträgt 97 %.

## Patentansprüche

1. Verfahren zur Herstellung von substituierten Phenolen der Formel 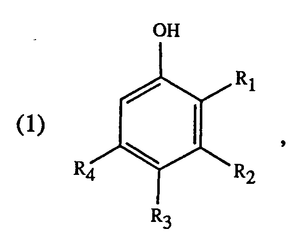 worin R₁, R₂, R₃ und R₄ unabhängig voneinander Wasserstoff, gegebenenfalls substituiertes Alkyl mit 1 bis 12 Kohlenstoffatomen oder gegebenenfalls substituiertes Alkenyl oder Alkinyl mit 2 bis 12 Kohlenstoffatomen sind, durch Erhitzen der Phenole der Formel 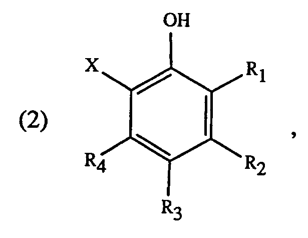 worin X sekundäres oder tertiäres Alkyl mit 3 bis 12 Kohlenstoffatomen ist, und R₁, R₂, R₃ und R₄ die angegebenen Bedeutungen haben, in Gegenwart eines sauren Katalysators, dadurch gekennzeichnet, dass man die Phenole der Formel (2) zur Abspaltung des Substituenten X mit Natriumhydrogensulfat als Katalysator, welcher nicht auf einem Trägermaterial fixiert ist, über ihren Schmelzpunkt erhitzt, die Schmelze abkühlen lässt und gegebenenfalls die Phenole der Formel (1) isoliert.

2. Verfahren nach Anspruch 1, dadurch gekennzeichnet, dass Phenole der Formel 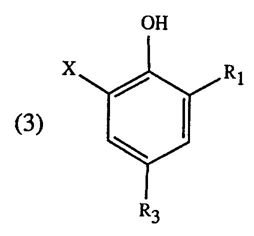 erhitzt werden, worin R₁, R₃ und X die angegebenen Bedeutungen haben.

3. Verfahren nach Anspruch 2, dadurch gekennzeichnet, dass Phenole der Formel (3) erhitzt werden, worin R₁ Wasserstoff, Alkyl mit 1 bis 12 Kohlenstoffatomen, Benzyl, Phenyläthyl oder Phenylpropyl, R₃ gegebenenfalls mit Phenyl, -CO₂Z, worin Z Wasserstoff, Alkyl mit 1 bis 18 Kohlenstoffatomen oder 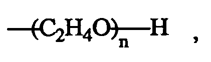 worin n 1 bis 12 ist, bedeutet, substituiertes Alkyl mit 1 bis 8 Kohlenstoffatomen und X tertiäres Alkyl mit 4 bis 9 Kohlenstoffatomen ist.

4. Verfahren nach Anspruch 3, dadurch gekennzeichnet, dass Phenole der Formel (3) erhitzt werden, worin R₃ Methyl, t-Butyl oder -CH₂CH₂CO₂Z ist, worin Z die in Anspruch 3 angegebene Bedeutung hat.

5. Verfahren nach Anspruch 1, dadurch gekennzeichnet, dass Phenole der Formel 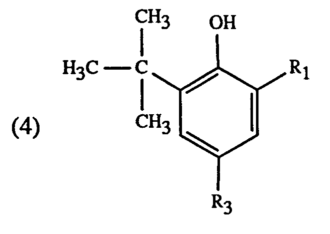 erhitzt werden, worin R₁ Wasserstoff oder Alkyl mit 1 bis 5 Kohlenstoffatomen und R₃ Alkyl mit 1 bis 4 Kohlenstoffatomen oder -CH₂CH₂CO₂CH₃ ist.

6. Verfahren nach Anspruch 5, dadurch gekennzeichnet, dass Phenole der Formel (4) erhitzt werden, worin R₁ Methyl oder t-Butyl und R₃ Methyl, t-Butyl oder -CH₂CH₂CO₂CH₃ ist.

## Claims

1. A process for the preparation of a substituted phenol of formula 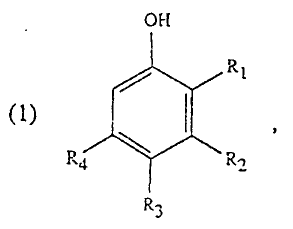 wherein R₁, R₂, R₃ and R₄ are each independently of one another hydrogen, unsubstituted or substituted alkyl of 1 to 12 carbon atoms or unsubstituted or substituted alkenyl or alkynyl of 2 to 12 carbon atoms, by heating a phenol of formula 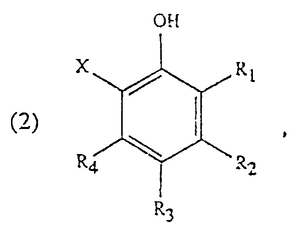 wherein X is secondary or tertiary alkyl of 3 to 12 carbon atoms, and R₁, R₂, R₃ and R₄ have the given meanings, in the presence of an acid catalyst, which process comprises heating said phenol of formula (2) to a temperature above its melting point to remove the substituent X, using sodium hydrogensulfate as catalyst, which is not fixed to a support material, allowing the melt to cool and, if desired, isolating the phenol of formula (1).

2. A process according to claim 1, which comprises heating a phenol of formula 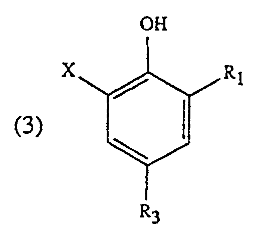 wherein R₁, R₃ and X have the given meanings.

3. A process according to claim 2, which comprises heating a phenol of formula (3), wherein R₁ is hydrogen, alkyl of 1 to 12 carbon atoms, benzyl, phenylethyl or phenylpropyl, R₃ is alkyl of 1 to 8 carbon atoms which is unsubstituted or substituted by phenyl, -CO₂Z, wherein Z is hydrogen, alkyl of 1 to 18 carbon atoms or 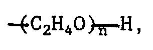 wherein n is 1 to 12, and X is tertiary alkyl of 4 to 9 carbon atoms.

4. A process according to claim 3, which comprises heating a phenol of formula (3), wherein R₃ is methyl, tert-butyl or -CH₂CH₂CO₂Z, wherein Z is as defined in claim 3.

5. A process according to claim 1, which comprises heating a phenol of formula 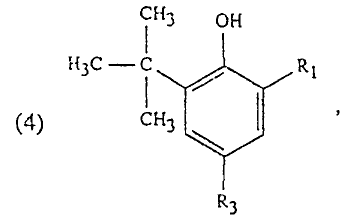 wherein R₁ is hydrogen or alkyl of 1 to 5 carbon atoms, and R₃ is alkyl of 1 to 4 carbon atoms or -CH₂CH₂CO₂CH₃.

6. A process according to claim 5, which comprises heating a phenol of formula (4), wherein R₁ is methyl or tert-butyl and R₃ is methyl, tert-butyl or -CH₂CH₂CO₂CH₃.

## Revendications

1. Procédé de préparation de phénols substitués de formule : 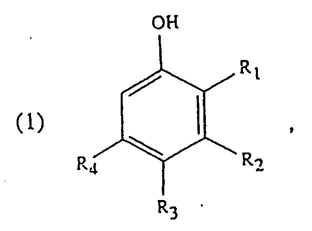 (dans laquelle R₁, R₂, R₃ et R₄ représentent chacun, indépendamment l'un de l'autre, un atome d'hydrogène, un reste alkyle éventuellement substitué comportant 1 à 12 atomes de carbone ou un reste alcényle ou alcynyle éventuellement substitué, comportant 2 à 12 atomes de carbone) par chauffage de phénols de formule : 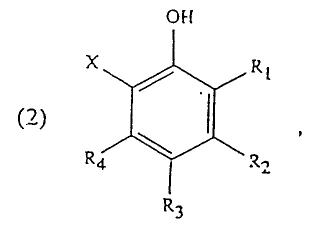 (dans laquelle X représente un reste alkyle secondaire ou tertiaire ayant 3 à 12 atomes de carbone, et R₁, R₂, R₃ et R₄ ont les sens indiqués) en présence d'un catalyseur acide, procédé caractérisé en ce qu'on chauffe au-delà de leur point de fusion les phénols de formule (2), pour en séparer par scission le substituant X, en présence d'hydrogéno sulfate de sodium comme catalyseur, lequel n'est pas fixé sur une matière de support, on laisse refroidir la masse fondue et éventuellement on isole les phénols de formule (1).

2. Procédé selon la revendication 1, caractérisé en ce que l'on chauffe des phénols de formule : 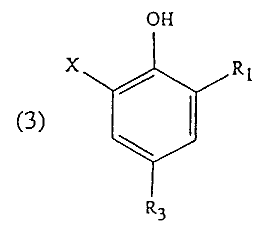 dans laquelle R₁, R₃ et X ont le sens indiqué.

3. Procédé selon la revendication 2, caractérisé en ce qu'on chauffe des phénols de formule (3), (dans laquelle R₁ représente un atome d'hydrogène, un reste alkyle ayant 1 à 12 atomes de carbone, benzyle, phényléthyle ou phénylpropyle; R₃ représente un reste alkyle substitué ayant 1 à 8 atomes de carbone, éventuellement substitué par un groupe phényle, -CO₂Z (dans lequel Z représente un atome d'hydrogène, un reste alkyle ayant 1 à 18 atomes de carbone ou -(C₂H₄O)ₙ-H , dans lequel n vaut 1 à 12), et X représente un reste alkyle tertiaire ayant 4 à 9 atomes de carbone.

4. Procédé selon la revendication 3 caractérisé en ce qu'on chauffe des phénols de formule (3), dans laquelle R₃ représente un groupe méthyle, t-butyle ou -CH₂CH₂CO₂Z, dans lequel Z a le sens indiqué à la revendication 3.

5. Procédé selon la revendication 1, caractérisé en ce qu'on chauffe des phénols de formule : 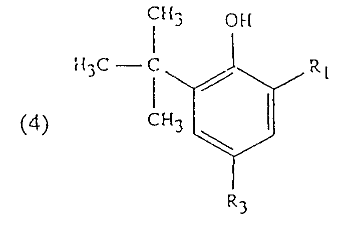 dans laquelle R₁ représente un atome d'hydrogène ou un reste alkyle ayant 1 à 5 atomes de carbone, et R₃ représente un reste alkyle ayant 1 à 4 atomes de carbone ou le reste -CH₂CH₂CO₂CH₃.

6. Procédé selon la revendication 5, caractérisé en ce qu'on chauffe des phénols de formule (4), dans laquelle R₁ représente un reste méthyle ou t-butyle et R₃ représente un reste méthyle, t-butyle ou -CH₂CH₂CO₂CH₃.
